# EUROPÄISCHE PATENTSCHRIFT

(11) **EP 2 615 978 B1**
(45) Veröffentlichungstag und Bekanntmachung des Hinweises auf die Patenterteilung: **20.12.2017**
(21) Anmeldenummer: 11758196.7
(22) Anmeldetag: 15.09.2011
(51) Int. Cl.: A61B 8/08, A61D 17/00

(54) **VORRICHTUNG ZUR TRÄCHTIGKEITSERKENNUNG**
DEVICE FOR DETECTING GESTATION
DISPOSITIF DE DÉTECTION DE GRAVIDITÉ

(30) Priorität: 15.09.2010 DE 202010012336 U
(43) Veröffentlichungstag der Anmeldung: 24.07.2013
(73) Patentinhaber: Big Dutchman International GmbH, 49377 Vechta-Calveslage (DE)
(72) Erfinder: FUCHS, Karsten, 49377 Vechta-Calveslage (DE); HOLLING, Daniel, 49377 Vechta-Calveslage (DE)
(74) Vertreter: Eisenführ Speiser
(86) Internationale Anmeldenummer: PCT/EP2011/066031
(87) Internationale Veröffentlichungsnummer: WO 2012/035111

(56) Entgegenhaltungen:
- EP-A2- 1 344 451
- BICHMAN M: "TRAECHTIGKEITSDIAGNOSE PER ULTRASCHALL", LANDTECHNIK, LANDWIRTSCHAFTSVERLAG, MUNSTER, DE, Bd. 52, Nr. 4, 1. August 1997 (1997-08-01) , Seite 196/197, XP000693099, ISSN: 0023-8082

## Beschreibung

Die Erfindung betrifft eine Vorrichtung zur Trächtigkeitserkennung bei Nutztieren gemäß dem unabhängigen Anspruch 1. Ein weiterer Aspekt der Erfindung ist ein Verfahren zur Trächtigkeitserkennung bei Nutztieren gemäß dem unabhängigen Anspruch 11. Vorrichtungen und Verfahren zur Trächtigkeitserkennung bei Nutztieren sind grundsätzlich bekannt und dienen dazu, festzustellen, ob ein Nutztier trächtig ist oder nicht. Es ist wünschenswert, diese Feststellung einerseits zu einem frühen Zeitpunkt der Trächtigkeit und andererseits mit einer hohen Sicherheit zu treffen. Beide Anforderungen sind für den Ablauf der Tieraufzucht und die Gesundheit des Muttertieres von prinzipieller Bedeutung.

Bekannte Vorrichtungen und Verfahren beruhen auf einer veterinärmedizinischen Untersuchung des Tieres und wenden hierzu Vorrichtungen und Verfahren an, die auch aus dem humanmedizinischen Bereich zur entsprechenden Diagnostik bekannt sind. Typischerweise erfolgt die Untersuchung mithilfe eines Ultraschallmessgerätes, welches eine zweidimensionale Bildaufnahme erzeugt, anhand derer ein Fachmann eine Beurteilung über die Trächtigkeit des Tieres treffen kann. Zu diesem Zweck wird ein Ultraschallmesskopf solcherart auf die Hautoberfläche des zu untersuchenden Tieres aufgelegt, dass der vom Ultraschallmesskopf erfasste Messbereich die Gebärmutter des Tieres erfasst und folglich ein oder mehrere darin befindliche Embryonen erfassen und bildlich wiedergeben kann. Um eine Aussage in der gewünschten sicheren Weise, zugleich aber frühen Phase treffen zu können, ist dieses bekannte Verfahren allerdings kostspielig, denn es erfordert die zeitlich engmaschige Untersuchung des Tieres mit einer kostspieligen Untersuchungsvorrichtung durch qualifiziertes Personal.

Eine vorbekannte Vorrichtung ist aus EP 0 386 503 B1 bekannt. Die vorbekannte Vorrichtung dient zur Feststellung der Trächtigkeit von weiblichen Großtieren und beschreibt hierzu ein Ultraschallgerät mit einer spezifischen Ausgestaltung der Stirnfläche des Schallkopfes. Die solcherart vorbekannte Technologie stellt zwar in bestimmten Anwendungsfällen, insbesondere bei behaarten Nutztieren, eine bessere Schallankopplung des Schallkopfes bereit und erlaubt somit auf Grundlage einer besseren Bildgebung auch eine zuverlässigere Beurteilung des Trächtigkeitszustandes. Allerdings wird durch diese vorbekannte Vorrichtung kein Vorteil hinsichtlich der Kosten der Untersuchungsweise und der Möglichkeit einer zeitlich engmaschigen Überprüfung erzielt.

Aus EP 0 173 837 ist ein weiteres Schallkopfsystem vorbekannt. Dieses System ist ebenfalls als Handgerät ausgeführt und erfordert die Untersuchung und Auswertung durch einen qualifizierten Fachmann. Auch mit diesem vorbekannten Gerät werden somit weder die Kosten für eine Untersuchung herabgesetzt noch die Möglichkeiten einer effizienten, zeitlich engmaschigen Überwachung geschaffen.

Aus DE 94 05 822 U ist ein weiteres Ultraschallgerät vorbekannt, welches eine teilautomatisierte Trächtigkeitserkennung anstrebt. Bei diesem Gerät wird eine durch einen qualifizierten Fachmann aufgenommene Ultraschall-Bildgebung automatisch ausgewertet und ein Signal für eine Trächtigkeit ausgegeben. Nachteilig an diesem Gerät ist, dass auch hier die Kosten bei einer zeitlich engmaschigen Überwachung nicht reduziert werden können. Zudem hat sich herausgestellt, dass die automatisierte Bildauswertung bei diesem Gerät nur unzuverlässige Ergebnisse liefert, wodurch eine sichere Aussage über eine bestehende Trächtigkeit mit dem System nicht getroffen werden kann.

Aus WO 2007/085073 ist eine weitere Ultraschalluntersuchungseinheit bekannt, welche auf Grundlage eines Dämpfungskoeffizienten des Ultraschallsignals anstrebt, eine bestehende Trächtigkeit eines Nutztieres zu erkennen. Auch das in diesem Stand der Technik beschriebene Verfahren greift jedoch auf eine kostspielige Vorrichtung zurück, die durch einen qualifizierten Fachmann bedient werden muss und stellt lediglich eine alternative oder ergänzende Beurteilungsgrundlage bereit. Nachteilig an diesem Stand der Technik ist demzufolge auch, dass eine Reduktion der Kosten mit dem Verfahren nicht gelingen kann, die Zuverlässigkeit der Aussage nur durch zusätzliche Maßnahmen erhöht wird, bei einer alternativen Beurteilung alleine aufgrund des Dämpfungskoeffizienten jedoch keine Steigerung der Zuverlässigkeit erzielt wird.

Aus EP 1 344 451 A2 ist eine Vorrichtung mit Messmitteln zum Messen einer Charakteristik von wenigstens einem Teil des Körpers eines Nutztieres bekannt. Die Vorrichtung ist mit einem Ultraschallmesskopf ausgerüstet, um den Fettgehalt von wenigstens einem Teil des Körpers zu bestimmen.

Eine Vorrichtung zur Trächtigkeitsuntersuchung an einem Nutztier gemäß dem Oberbegriff des Anspruchs 1 ist aus Bichman M: "Trächtigkeitsdiagnose per Ultraschall", XP000693099, ISSN: 0023-8082, bekannt.

Es ist vor diesem Hintergrund Aufgabe der Erfindung, eine Vorrichtung zur Trächtigkeitsuntersuchung bereitzustellen, welche bei insgesamt verringerten Kosten eine zumindest ebenso hohe Zuverlässigkeit der Trächtigkeitserkennung bereitstellt wie der Stand der Technik oder bei zumindest gleichbleibenden Kosten eine höhere Zuverlässigkeit in der Trächtigkeitserkennung bereitstellt.

Diese Aufgabe wird erfindungsgemäß durch eine Vorrichtung zur Trächtigkeitsuntersuchung an einem Nutztier gemäß dem unabhängigen Anspruch 1 gelöst. Der Erfindung liegt einerseits die Erkenntnis zugrunde, dass durch eine reproduzierbare relative Positionierung eines Ultraschallmesskopfes zu einem zu untersuchenden Nutztier sowohl eine Vereinfachung des Untersuchungsvorgangs als auch eine Steigerung der Zuverlässigkeit des Ergebnisses der Trächtigkeitsuntersuchung möglich ist. Dabei spielen mehrere Faktoren zusammen, die dieses Ergebnis erreichen lassen. Zum einen wird durch die Aufnahmevorrichtung und die am beweglichen Gestell angeordnete Halterung für den Ultraschallmesskopf das Prozedere der Untersuchung vereinfacht, da einerseits das Nutztier in eine definierte Position und Körperhaltung gebracht werden kann, andererseits auf dieser Grundlage sowohl der Ort als auch die Ausrichtung des vom Ultraschallmesskopf ausgesendeten Ultraschallsignals für jede eine Reihe von Messungen reproduzierbar in Bezug auf das jeweilige Tier eingestellt werden können. Das heißt, sowohl mehrere Messungen an einem Tier als auch an mehreren Tieren werden mit gleicher Position und Ausrichtung des Schallmesskopfes vorgenommen. Dies ermöglicht es, dass die Untersuchung durch Personal durchgeführt wird, welches keine veterinärmedizinische Grundausbildung oder eine spezifisch auf die Durchführung von Trächtigkeitsuntersuchungen gerichtete Schulung absolviert hat. Des Weiteren wird durch die erfindungsgemäße Vorrichtung ein Messergebnis produziert, welches aufgrund seiner stets konstanten Positionierung und Ausrichtung des Bildergebnisses einer automatisierten Auswertung der Bilddaten in wesentlich besserer Weise zugänglich ist als Messdaten, die durch ein von qualifiziertem Personal handgeführtes Gerät gewonnen werden. Die Ursache liegt hierbei darin, dass nach Erkenntnis der Erfinder übliche Auswertungs- und Filtermethoden in solchen Bildauswertungen automatisiert werden können, wenn Grundlage der Auswertung ein stets wiederkehrendes typisches Bild mit typischen wiederkehrenden Artefakten ist. Gerade dies wird aber durch eine manuell von qualifiziertem Personal optimierte Bildaufnahme in der Regel nicht gewonnen, da hier auf der Suche nach dem besten Bild mit dem kleinsten Artefakt unregelmäßig immer wieder von Bild zu Bild unterschiedliche Artefakte erzeugt werden, die nicht zuverlässig mit einem automatisierten Auswertungsverfahren auszufiltern wären. Schließlich ist ein weiterer Grund für den erfindungsgemäß erzielten Vorteil die Möglichkeit, die Trächtigkeitsuntersuchung im Zuge einer ohnehin erfolgenden Vereinzelung von Tieren innerhalb des Stalles, beispielsweise bei der Fütterung der Tiere, vorzunehmen, was eine zeitlich engmaschige Kontrolle ermöglicht, ohne hierdurch maßgeblichen zusätzlichen Aufwand in apparativer Hinsicht oder im Hinblick auf den Tagesablauf des Nutztieres betreiben zu müssen.

Im Sinne der erfindungsgemäßen Vorrichtung soll unter einer Aufnahmevorrichtung eine ein- oder mehrteilige Vorrichtung verstanden werden, welche einen dreidimensionalen Raum umgrenzt, in dem genau ein Nutztier Platz findet. Dabei ist es bevorzugt so, dass durch die Aufnahmevorrichtung sowohl die Position als auch die Haltung des Tieres in möglichst genauer Weise vorbestimmt wird. Diese Position und Haltung sollte bei Nutzung der Aufnahmevorrichtung durch mehrere Tiere reproduzierbar gleich sein, um hierdurch eine zuverlässige Positionierung der Halterung mit dem Ultraschallmesskopf durch das Gestell zu ermöglichen. Ein Beispiel für eine solche Aufnahmevorrichtung ist beispielsweise ein von zwei Seitenwänden begrenzter Gang, in den ein Nutztier eintreten kann, um an dessen Stirnseite eine Futterstelle vorzufinden und dort Futter aufzunehmen. Durch die Seitenwände wird das Tier hierbei in seitlicher Hinsicht positioniert und durch die Futterstelle wird das Tier in Längsrichtung des Ganges positioniert, wobei durch den Vorgang der Futteraufnahme eine hierfür typische Haltung des Tieres sichergestellt wird, d.h. ein Sitzen oder Liegen des Tieres typischerweise vermieden wird.

Die erfindungsgemäß vorgesehene Halterung für den Ultraschallmesskopf soll es ermöglichen, dass ein Ultraschallmesskopf darin vorzugsweise lösbar befestigt ist. Je nach Ausführung des Ultraschallmesskopfes kann diese Halterung unterschiedlich ausgeführt sein, beispielsweise in Form einer oder mehrerer Gewindebohrungen, in Gestalt einer form- und/oder kraftschlüssigen Halterung, in welche der Ultraschallmesskopf beispielsweise eingeklemmt werden kann oder in noch anderer Bauweise.

Weiterer Bestandteil der erfindungsgemäßen Vorrichtung ist ein Gestell, an dem diese Halterung befestigt ist und das relativ zu der Aufnahmevorrichtung beweglich ist. Dieses Gestell kann in einfacher Weise nur in Bezug auf eine Raumachse beweglich sein, sei es durch eine translatorische Bewegung oder eine rotatorische Bewegung, um hierdurch die Halterung mit dem daran angeordneten Ultraschallmesskopf an den Körper des Nutztieres heranführen zu können. Insbesondere ist es bevorzugt, das Gestell an der Aufnahmevorrichtung zu befestigen und eine relative Beweglichkeit durch eine Schwenkachse oder eine Linearführung oder mehrere solcher Schwenkachsen oder Linearführungen und Kombinationen daraus bereitzustellen. Dabei ist grundsätzlich zu verstehen, dass im Hinblick auf die Anzahl der Freiheitsgrade, die diese Beweglichkeit beinhaltet, die notwendige Anzahl für eine zuverlässige Daten liefernde Positionierung des Ultraschallmesskopfes sichergestellt werden sollte und darüber hinausgehende Freiheitsgrade vermieden werden sollten. Bevorzugte Ausführungsformen hierzu sind in den nachfolgenden Ansprüchen ausgeführt.

Schließlich ist erfindungsgemäß eine Datenauswerteeinrichtung vorgesehen, die die Daten durch signaltechnische Übertragung erhält, die vom Ultraschallmesskopf erfasst werden. Diese Datenauswerteeinheit kann vorzugsweise kabelgebunden, gegebenenfalls aber auch kabellos mit dem Ultraschallmesskopf verbunden sein und ist so ausgebildet, dass eine automatisierte Auswertung der Ultraschallbilder, beispielsweise in Form einer Grauwertanalyse, erfolgen kann, um aus dem erhaltenen Ultraschallbild eine Messaussage im Hinblick auf die Trächtigkeit des Tieres zu erhalten.

Gemäß einer ersten bevorzugten Ausführungsform ist vorgesehen, dass die Aufnahmevorrichtung zwei einen Aufenthaltsraum des Nutztieres während der Untersuchung begrenzende Seitenwände aufweist und das Gestell eine Brücke aufweist, die ausgebildet ist, um entlang dieser Seitenwände horizontal bewegt zu werden, insbesondere mittels einer an den Seitenwänden befestigten Führungseinrichtung.

Mit dieser Ausgestaltung wird das Gestell in spezieller Zusammenwirkung mit der Aufnahmevorrichtung beweglich ausgeführt, um hierdurch einerseits das zu untersuchende Nutztier definiert zu positionieren, andererseits die Möglichkeit zu schaffen, die Halterung des Ultraschallmesskopfes hinsichtlich der variablen Körperlänge des Nutztieres durch Bewegung der Brücke entlang der Seitenwände positionieren zu können. Die Brücke ist dabei vorzugsweise auf den oberen Kanten der Seitenwände gelagert und kann auf diese Weise insbesondere aus einer Ruheposition, in welcher der Eintritt des Tieres in die Aufnahmevorrichtung nicht behindert wird, in der horizontalen Richtung in eine Messposition bewegt werden. Unter Seitenwänden ist hierbei einerseits eine geschlossene Wandfläche zu verstehen, in anderen Ausführungsformen können die Seitenwände auch durch Streben, Gitter oder dgl. bereitgestellt werden. Die Brücke erstreckt sich dabei vorzugsweise von einer Seitenwand zur anderen Seitenwand in einer Richtung senkrecht zur den durch die Seitenwände definierten Ebenen.

Noch weiter ist es bevorzugt, dass die Aufnahmevorrichtung zwei einen Aufenthaltsraum des Nutztieres während der Untersuchung begrenzende Seitenwände aufweist und das Gestell eine Vertikalstrebe aufweist, mit der die Halterung für eine vertikale Beweglichkeit des Ultraschallkopfs verbunden ist, insbesondere solcherart, dass die Vertikalstrebe mit einer horizontal entlang der Seitenwände beweglichen Brücke verbunden ist und die Vertikalstrebe gemeinsam mit dem Ultraschallkopf vertikal zu dieser Brücke beweglich ist oder der Ultraschallkopf entlang der Vertikalstrebe vertikal beweglich ist. Eine solche Ausgestaltung des Gestells mit Vertikalstreben ermöglicht die Positionierung der Halterung in vertikaler Richtung, um hierdurch die Halterung auf unterschiedliche Höhen verfahren zu können und folglich bei unterschiedlichen Tierhöhen eine exakte Positionierung des Ultraschallmesskopfs an einer gewünschten Messposition zu erzielen. Prinzipiell kann diese vertikale Beweglichkeit erreicht werden, indem der Ultraschallkopf an der Vertikalstrebe angeordnet ist und die Vertikalstrebe selbst vertikal verschoben werden kann oder indem die Vertikalstrebe selbst unbeweglich ist, die Halterung für den Ultraschallkopf aber entlang der Vertikalstrebe verschoben werden kann. Bei der besonders bevorzugten Ausführungsform eines Gestells mit Brücke und Vertikalstrebe wird eine Beweglichkeit der Halterung für den Ultraschallkopf in zwei Achsrichtungen bereitgestellt, wodurch die Halterung einerseits in Bezug auf unterschiedliche Längen der zu untersuchenden Nutztiere, andererseits auf unterschiedliche Höhen der zu untersuchenden Nutztiere hinsichtlich der daraus resultierenden Messpositionierung bewegt werden kann. Auf diese Weise wird sichergestellt, dass Nutztiere unterschiedlicher Länge und Höhe mit der erfindungsgemäßen Vorrichtung zuverlässig untersucht werden können.

Noch weiter ist es bevorzugt, dass die Halterung am Ende einer mehrgliedrigen Gliederkette befestigt ist, in der jeweils zwei benachbarte Kettenglieder um jeweils eine Kettengliedachse relativ zueinander verschwenkbar sind, die parallel zu der durch den Aufenthaltsraum definierten Längsachse des Nutztieres, insbesondere horizontal und parallel zu den Aufenthaltsraum begrenzenden Seitenwänden liegt. Diese Fortbildung befasst sich mit einer spezifischen Problematik im Zusammenhang mit einer voll- oder teilautomatisierten Untersuchung von Nutztieren mit diagnostischen Geräten. Angestrebt wird bei der hier eingesetzten Ultraschallmesstechnik eine flächige Anlage des Ultraschallmesskopfes, die unter einem bestimmten Anpressdruck erfolgen soll. Die Neigung der Körperoberfläche des Nutztieres im Bereich der Messposition und benachbart hierzu ist dabei regelmäßig nicht zuverlässig vorhersagbar und unter anderem von der Haltung des Tieres, seiner Körperfülle und dgl. abhängig. Das Anlegen eines Ultraschallmesskopfes muss in einer Art und Weise erfolgen, die das Tier nicht durch den Kontakt erschreckt und zu Schreckreaktionen des Tieres führen könnte, um eine zuverlässige Messung zu erreichen. Es hat sich gemäß dieser erfindungsgemäßen Fortbildung herausgestellt, dass durch Anordnen der Halterung am Ende einer Gliederkette ein definiertes Anlegen des in der Halterung befindlichen Ultraschallmesskopfes an die Körperoberfläche des Tieres erzielt werden kann. Der besondere Vorteil liegt hierbei darin, dass keine starren Elemente eingesetzt werden, um die Halterung in dem relevanten, körpernahen Bereich des Tieres am Gestell zu führen und folglich keine lokalen Druckkräfte auf den Körper des Tieres ausgeübt werden können, welche das Tier erschrecken könnten. Die Gliederkette ermöglicht eine definierte Führung der Halterung und zugleich eine nahezu druckfreie Anlage des Gestells am Körper des Nutztieres im Bereich um die Halterung, wodurch eine zuverlässige Messung begünstigt wird. Dies wird insbesondere dann erreicht, wenn die Gliederkette solcherart hinsichtlich ihrer Kettenachsen ausgerichtet ist, dass sie sich bei vertikaler Längserstreckung um den Körper des Tieres schmiegen kann und hierdurch die gewünschte druckarme Anlage am Körper des Tieres erzielt wird.

Dabei ist es besonders bevorzugt, wenn die Gliederkette um zumindest eine Mehrzahl der Kettengliedachsen mittels eines Aktuators verschwenkbar ist, vorzugsweise mittels eines innerhalb der Gliederkette beabstandet von den Kettengliedachsen geführtem Zugseil, das mit einer aktuatorbetätigten Zugvorrichtung verbunden ist. Grundsätzlich kann die Gliederkette durch unterschiedliche Aktuatoren betätigt werden, insbesondere können hier auch Aktuatoren zum Einsatz kommen, die außerhalb der Gliederkette angeordnet sind und beispielsweise nur auf die Halterung einwirken, um diese an der Körperoberfläche des Nutztieres zu positionieren. Diese Aktuatoren können sich von einem Gestellpunkt zu der Gliederkette erstrecken. Eine besonders vorteilhafte Beweglichkeit der Gliederkette wird jedoch erreicht, wenn ein Zugseil dazu eingesetzt wird, um die Kettenglieder gegeneinander zu verschwenken. Dies kann insbesondere wirksam erreicht werden, wenn das Zugseil innerhalb der Kette verlegt ist und durch eine entsprechende Ausgestaltung der einzelnen Kettenglieder darin geführt wird. Ein solcherart geführtes Zugseil kann, wenn es beabstandet von zumindest einigen Kettengliedachsen verläuft, eine variable, an einer unregelmäßig gekrümmtem Oberfläche sich anlegende Krümmung der Kette hervorrufen, wenn es gespannt wird. Diese Spannung des Zugseils kann aktuatorbetätigt erfolgen, wobei hierunter eine entsprechende manuelle Spannmöglichkeit des Zugseils mittels einer beispielsweise hebelförmigen Spannvorrichtung zu verstehen ist oder eine durch einen elektrischen, pneumatischen oder in anderer Weise angetriebenen Aktuator bewirkte Spannung des Zugseils.

Gemäß einer weiteren bevorzugten Ausführungsform ist vorgesehen, dass die Halterung des Ultraschallmesskopfs mit einem Aktuator gekoppelt ist zur Positionierung des Ultraschallmesskopfs an einer vorbestimmten Körperstelle des Nutztieres, das Gestell eine relative Beweglichkeit der Halterung des Ultraschallmesskopfs in zumindest einer Achse bereitstellt und eine Steuerung zur aktiven Positionierung und passiven Nachführung eines in der Halterung aufgenommenen Ultraschallmesskopfes bereitgestellt ist, welche ausgebildet ist, um die Halterung in einem Positionierungsmodus mittels eines oder mehreren Aktuatoren des Gestells so in Bezug auf die Aufnahmevorrichtung aktiv örtlich zu positionieren, wobei das Gestell ausschließlich aktuatorbetätigte Bewegungen der Halterung ausführt, und die Halterung in einem Messmodus in zumindest einer Achse so frei beweglich durch das Gestell so zu führen, dass die Halterung Bewegungen des Nutztieres passiv folgen kann.

Diese Ausführungsform erreicht eine exakte Positionierung der Halterung an dem Nutztier, indem zu diesem Zweck im Positionierungsmodus eine insgesamt steife Gestellkonfiguration bereitgestellt wird, in welcher die Halterung durch Bewegungen innerhalb des Gestells in einer oder mehreren Achsen auf die gewünschte Messposition bewegt werden kann. Insbesondere kann unter dieser aktiven Positionierung der Halterung eine horizontale Verschiebung einer Brücke des Gestells, eine vertikale Verschiebung der Halterung mittels oder entlang einer Vertikalstrebe und / oder ein seitliches Anlegen der Halterung an die Körperoberfläche des Nutztieres, beispielsweise mittels einer zugseilbeaufschlagten Gliederkette verstanden werden. Nachdem der Messkopf an der gewünschten Messposition auf die Körperoberfläche des Nutztieres aufgelegt wurde, tritt durch die dort erreichte Anpresskraft eine gewisse Haftung auf. Um nach diesem Anlegen die Messung über einen bestimmten Zeitraum durchführen zu können und nicht durch Bewegungen des Nutztieres beeinträchtigen zu lassen, ist es dann vorteilhaft, wenn das Gestell aus der steifen Gestellkonfiguration in eine Gestellkonfiguration gebracht wird, in der es in zumindest einer Achse solchen Bewegungen des Nutztieres folgen kann. So kann insbesondere vorgesehen sein, dass das Gestell eine oberhalb des Nutztieres liegende horizontale Achse aufweist, die sich quer zur Längsachse des Nutztieres erstreckt und es ermöglicht, dass die Halterung an vertikal von dieser Achse herabreichenden Streben angeordnet ist und um diese Achse verschwenkt werden kann und folglich Bewegungen des Nutztieres in seiner Längsrichtung folgen kann. Dabei ist zu verstehen, dass der Positionierungsmodus auch erzielt werden kann, wenn durch die Trägheit des Gestells eine definierte Position der Halterung beispielsweise schwerkraftbedingt sich auch nach Verfahren von einzelnen Elementen des Gestells reproduzierbar einstellt, ohne dass eine bewegliche Achse hierzu blockiert werden müsste. Bei einer solchen Ausgestaltung des Gestells kann folglich auf eine aktive Blockade und Freigabe einer Achse des Gestells verzichtet werden, ohne hierbei den Vorteil einer exakten Positionierung und Nachführung der Halterung aufgeben zu müssen.

Gemäß einer weiteren bevorzugten Ausführungsform ist eine Benetzungsvorrichtung vorgesehen zur Zufuhr eines pastösen oder flüssigen Schallkopplungsmediums auf die Sende- und Empfangsfläche des Ultraschallkopfs, die Benetzungsvorrichtung umfassend einen Vorratsbehälter für das Schallkopplungsmedium, eine Verbindungsleitung zur Leitung des Schallkopplungsmediums von dem Vorratsbehälter zu einer Austrittsöffnung, welche so angeordnet ist, dass aus der Austrittsöffnung austretendes Schallkopplungsmedium auf die Sende- und Empfangsfläche des Ultraschallkopfs aufgetragen wird, und eine Fördervorrichtung zum Fördern des Schallkopplungsmediums von dem Vorratsbehälter zu der Austrittsöffnung. Mit dieser Fortbildung wird eine automatisierte Benetzung des Messkopfes mit einem Kopplungsmedium erzielt, wodurch die Effizienz und Zuverlässigkeit der erfindungsgemäßen Vorrichtung weiter erhöht wird. Dabei kann vorgesehen sein, dass die Benetzungsvorrichtung unmittelbar an der Halterung angeordnet ist, wodurch es ermöglicht wird, das Kopplungsmedium auf den Ultraschallmesskopf unmittelbar in jeder Position des Gestells aufzutragen. In einer anderen Ausgestaltung ist die Benetzungsvorrichtung so an dem Gestell oder der Aufnahmevorrichtung angeordnet, dass der Ultraschallmesskopf mittels der Halterung vor einer Messung an die Austrittsöffnung für das Kopplungsmedium herangeführt werden kann, dort benetzt wird und dann von der Benetzungsvorrichtung wieder weggeführt wird, um die Messung durchzuführen. Insbesondere ist es bevorzugt, wenn die erfindungsgemäße Vorrichtung solcherart ausgeführt ist, dass die Aufnahmevorrichtung zwei einen Aufenthaltsraum des Nutztieres während der Untersuchung begrenzende Seitenwände aufweist und das Gestell eine Brücke aufweist, die ausgebildet ist, um entlang dieser Seitenwände horizontal bewegt zu werden, eine Vertikalstrebe mit der Brücke verbunden ist, die Halterung für den Ultraschallmesskopf am Ende einer mehrgliedrigen Gliederkette befestigt ist, in der jeweils zwei benachbarte Kettenglieder um jeweils eine Kettengliedachse relativ zueinander verschwenkbar sind, die parallel zu der durch den Aufenthaltsraum definierten Längsachse des Nutztieres, insbesondere horizontal und parallel zu den den Aufenthaltsraum begrenzenden Seitenwänden verläuft, die mehrgliedrige Kette an der Vertikalstrebe in der Höhe verschieblich und um eine horizontale, quer zur Längsachse des Aufenthaltsraumes verlaufende Nachführachse verschwenkbar gelagert ist, und die Nachführachse vorzugsweise mittels eines Positionier- und Nachführmechanismus in einem Positionierungsmodus arretierbar ist und in einem Messmodus frei bewegbar ist. Mit dieser Ausgestaltung wird eine Beweglichkeit des Gestells bereitgestellt, die es ermöglicht, die Halterung an eine Messposition auf der Körperoberfläche eines Nutztieres in einem definierten Winkel in Bezug auf die Horizontale heranzuführen und hierbei typische anatomische Größenvariationen der Nutztiere zu berücksichtigen. Insbesondere wird es durch diese Ausgestaltung des Gestells ermöglicht, zahlreiche für die Positionierung erforderliche Bewegungen der Halterung auszuführen, bevor die Halterung oder andere Teile des Gestells in Kontakt mit dem Nutztier treten und erst in einem letzten Schritt durch Anlegen der Gliederkette an die Körperoberfläche des Tieres einen druckarmen Kontakt zwischen dem Gestell und dem Ultraschallmesskopf mit der Körperoberfläche des Tieres zu erzeugen. Die erfindungsgemäße Vorrichtung weist auf eine zwei- oder dreidimensionale Bilderfassungseinrichtung zur Erfassung der Kontur oder eines Ausschnittes der Kontur des Nutztieres, eine mit der Bilderfassungseinrichtung signaltechnisch verbundene Bildauswertungseinheit, welche ausgebildet ist, um anhand der erfassten Kontur bzw. des erfassten Konturausschnitts einen Messpunkt oder Messbereich zu bestimmen, eine mit der Bildauswertungseinheit signaltechnisch verbundene Messkopfhalterungssteuereinheit, welche mit einem oder mehreren Aktuatoren zur Bewegung der Halterung für den Ultraschallmesskopf signaltechnisch verbunden ist und ausgebildet ist, um die Halterung so zu bewegen, dass ein darin aufgenommener Ultraschallmesskopf an den Messpunkt bzw. in den Messbereich geführt wird. Diese Fortbildung ermöglicht es, die Positionierung des Messkopfes auf der Körperoberfläche des Tieres und gegebenenfalls auch die Ausrichtung des Messkopfes zu automatisieren, indem die hierfür notwendigen anatomischen Abmessungen des Tieres durch eine Bilderfassung ermittelt werden. Dabei ist zu verstehen, dass nicht notwendigerweise eine vollständige anatomische Erfassung des Nutztieres erforderlich ist, um die notwendigen Daten für eine Positionierung zu ermitteln. Stattdessen kann oftmals aufgrund einzelner, ermittelter anatomischer Abmessungen, wie beispielsweise der Gesamtlänge des Tieres und der Breite des Tieres an einer bestimmten Stelle aufgrund von Erfahrungswerten, die in der Bildauswertungseinheit abgespeichert sind, auf die für eine Positionierung des Ultraschallmesskopfes relevanten anatomischen Abmessungen geschlossen werden. In der Regel ist folglich lediglich eine zweidimensionale Bilderfassung erforderlich, um eine ausreichend genaue Berechnung der Positionierungsdaten zu erreichen.

Dabei ist zu verstehen, dass die Bildauswertungseinheit auch ausgebildet sein kann, um darin eine solche anatomische Erfassung und Berechnung auch unter Zuweisung einer das Nutztier charakterisierenden Kennung abzuspeichern, um auf diese Daten bei einer nachfolgenden Messung wieder zurückgreifen zu können.

Weiterhin ist zu verstehen, dass die zwei- oder dreidimensionale Bilderfassung nicht nur dazu dient, um anatomische Abmessungen des Nutztieres zu erfassen, sondern auch dazu dienen kann, um die Position und Haltung des Tieres innerhalb der Aufnahmevorrichtung zu ermitteln. Oftmals ist es nicht adäquat oder nicht erreichbar, dass ein Nutztier in der Aufnahmevorrichtung so hinsichtlich seiner Position und Haltung definiert und fixiert wird, dass reproduzierbar bei Kenntnis der Messposition relativ am Körper eine zuverlässige Positionierung des Ultraschallmesskopfes daraus erfolgen kann. Stattdessen kann die erfindungsgemäße Bilderfassungseinrichtung eingesetzt werden, um Position und Haltung des Tieres durch eine oder mehrere Bildaufnahmen zu ermitteln, die Positionierung hiernach aufgrund einer Auswertung der solcherart erfassten Bilder erfolgen, um dann nach Anlegen des Ultraschallmesskopfes an das Tier diesen durch aktive oder passive Nachführung in der Messposition während der Messung zu halten.

Schließlich kann die erfindungsgemäße Vorrichtung noch weitergebildet werden durch eine an der Halterung angeordnete horizontale Schwenkachse, mittels derer die Halterung um eine horizontale und parallel zur Längsachse des Tieres liegende Achse verschwenkt werden kann und weiter vorzugsweise gekennzeichnet durch einen zwischen Gestell und Halterung mechanisch gekoppelten Schwenkaktuator, mittels dem die Halterung um diese horizontale Schwenkachse verschwenkbar ist. Mit dieser Fortbildung wird eine definierte Beweglichkeit der Halterung bereitgestellt, die es ermöglicht, den Messwinkel des Ultraschallmesskopfes in Bezug auf die Horizontale zu verändern. Diese Messrichtungsänderung hat sich erfindungsgemäß als bedeutsamer Einflussfaktor auf eine erfolgreiche Messung und zuverlässige Aussage zur Trächtigkeit herausgestellt. Insbesondere ist es für eine solche Aussage bevorzugt, wenn die Halterung um diese horizontal liegende Längsachse verschwenkt werden kann, um mehrere Messungen aufeinander folgend unter unterschiedlichen Winkeln zur Horizontalen durchführen zu können. Diese Messabfolge kann dann einer Auswertung zugeführt werden, wobei wahlweise alle Bilder ausgewertet werden oder aus den Bildern ein oder einzelne relevante Bilder ausgewählt und dann ausschließlich diese ausgewertet werden können.

Ein weiterer Aspekt der Erfindung ist ein Verfahren zur Trächtigkeitsuntersuchung an einem Nutztier gemäß dem unabhängigen Anspruch 11, umfassend die Schritte: Aufnehmen des Nutztieres in einem umgrenzten Aufenthaltsraum, Vermessen der Tierkontur mittels einer zwei- oder dreidimensionalen Bilderfassungseinrichtung, Auswerten der Tierkonturdaten und Bestimmen eines Messpunktes für eine Ultraschalluntersuchung anhand eines Vergleichs der Tierkonturdaten mit gespeicherten anatomischen Daten, Heranführen eines in einer Halterung gehaltenen Ultraschallmesskopfs an den Messpunkt durch Bewegen der Halterung mittels eines Gestells relativ zu dem Aufenthaltsraum, Erfassen von Messdaten mittels des Ultraschallmesskopfs, Auswerten der Messdaten durch Bilderkennung von Merkmalen die zur Differenzierung einer Trächtigkeit des Nutztieres herangezogen werden. Mit diesem Verfahren wird eine effiziente und vollständig oder teilweise automatisierte Trächtigkeitsuntersuchung an Nutztieren bereitgestellt, welche zuverlässige Ergebnisse liefert.

Das Verfahren kann nach den Ansprüchen 12 bis 15 fortgebildet werden. Hinsichtlich der spezifischen Ausgestaltung dieser Verfahrensfortbildungen und den hierdurch erzielten Vorteilen wird auf die voranstehende Beschreibung der entsprechend ausgebildeten Vorrichtungen und deren Fortbildungen Bezug genommen.

Eine bevorzugte Ausführungsform wird anhand der beiligenden Figuren erläutert. Es zeigen:
Fig. 1 eine Frontalansicht einer erfindungsgemäßen Trächtigkeitsuntersuchungsvorrichtung mit einem schematisch abgebildeten Nutztier,
Fig. 2 eine Seitenansicht der Vorrichtung gemäß Fig. 1,
Fig. 3 eine Draufsicht auf die Vorrichtung gemäß Fig. 1,
Fig. 4 eine perspektivische Ansicht von schräg links oben der erfindungsgemäßen Trächtigkeitsuntersuchungsvorrichtung,
Fig. 5 eine Ansicht gemäß Fig. 4 von schräg rechts vorne oben,
Fig. 6 eine perspektivische Detailansicht der Ausführungsform gemäß Fig. 4,
Fig. 7 eine seitliche Detailansicht der Ausführungsform gemäß Fig. 4,
Fig. 8 eine perspektivische Detailansicht einer Benetzungsvorrichtung für ein Kopplungsmedium der Ausführungsform gemäß Fig. 4,
Fig. 9 eine perspektivische Frontalansicht einer Halterung mit eingesetztem Schallmesskopf der Ausführungsform gemäß Fig. 4,
Fig. 10 eine perspektivische Detailansicht der Benetzungsvorrichtung gemäß Fig. 8, und
Fig. 11 eine Gesamtansicht der Benetzungsvorrichtung.

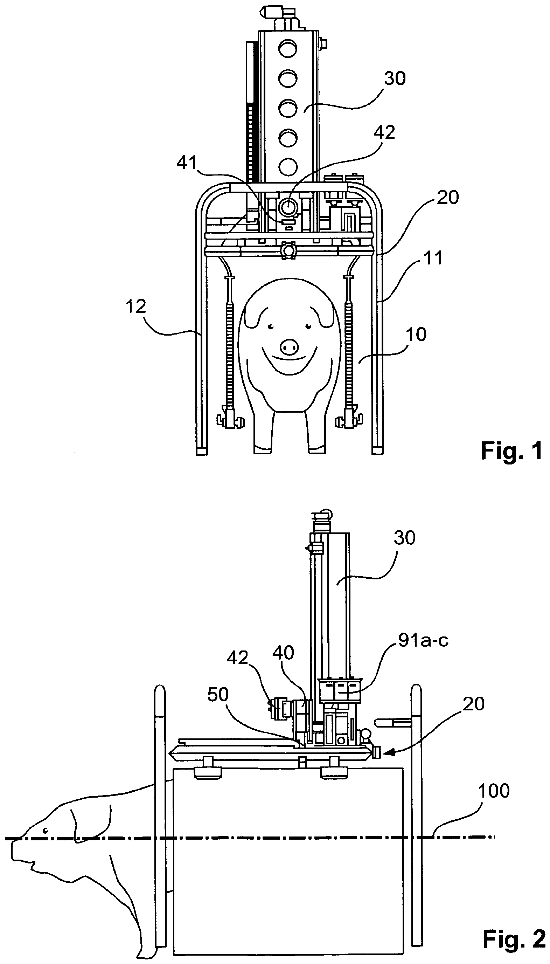

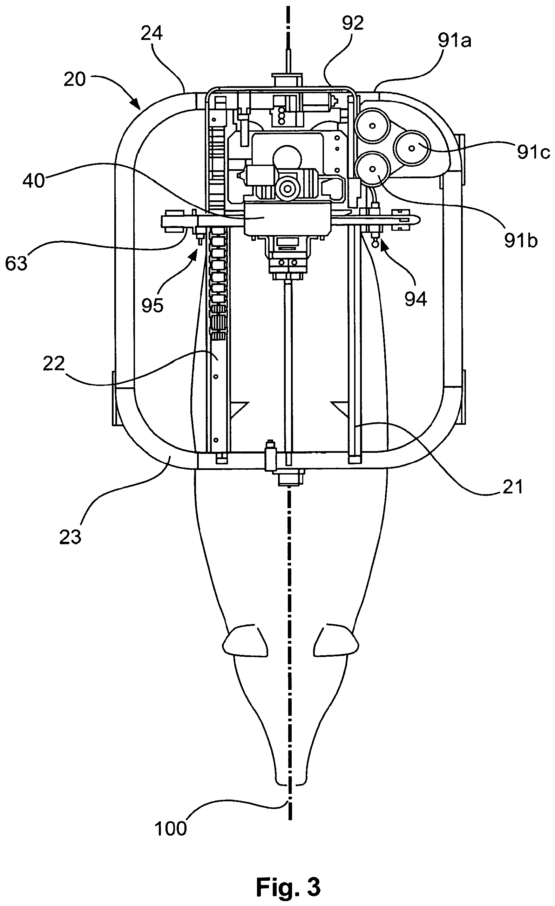

Bezug nehmend auf die Figuren 1 bis 3 ist erkennbar, dass bei der erfindungsgemäßen Vorrichtung ein Aufenthaltsbereich 10 durch Seitenwände 11, 12 begrenzt wird. Der Aufenthaltsraum definiert eine Mittellängsachse 100. In dem Aufenthaltsbereich 10 kann ein Nutztier, hier eine Sau, stehend Platz finden. Es ist zu verstehen, dass die Standposition des Nutztieres durch eine vorder- und rückseitige Begrenzung weiter in dem Aufenthaltsbereich definiert werden kann, um auf diese Weise sicherzustellen, dass das Nutztier an einer bestimmten Position in Bezug auf die Längsrichtung 100 des Aufenthaltsbereichs steht.

Oberhalb des Aufenthaltsbereichs ist mittels vier Streben auf den beiden Seitenwänden aufgesetzt ein rechteckiger Rahmen mit abgerundeten Ecken. Der Rahmen 20 ist ortsfest auf den Seitenwänden befestigt, kann jedoch für eine Grundeinstellung entlang der Seitenwände horizontal verschoben werden und dann in einer für die nachfolgenden Messungen im Wesentlichen passenden Position fixiert werden.

Zwei Längsstreben 21, 22 erstrecken sich parallel und symmetrisch zur Mittellängsachse 100 des Rahmens 20 von einer vorderen Rahmenstrebe 23 zu einer hinteren Rahmenstrebe 24 und sind fest mit diesen beiden Rahmenstreben verschweißt.

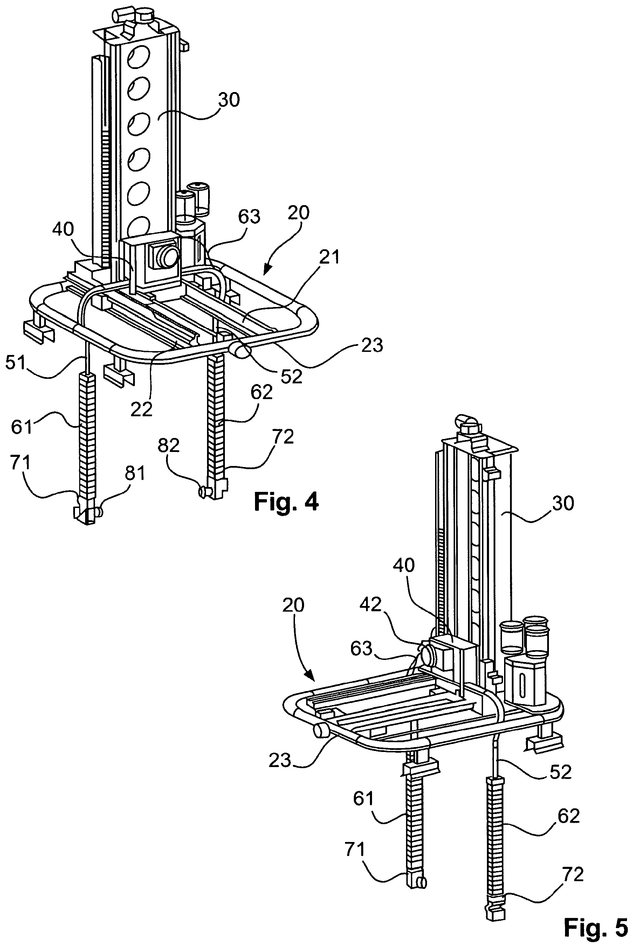

Bezug nehmend weiterhin auf die Figuren 4 und 5, welche die erfindungsgemäße Trächtigkeitsuntersuchungsvorrichtung ohne die Seitenwände zeigen, ist erkennbar, dass eine Vertikalstrebe 30 oberhalb der Längsstreben 21, 22 angeordnet ist und sich in vertikaler Richtung erstreckt. Die Vertikalstrebe 30 ist horizontal entlang der Längsstreben 21, 22 verschiebbar.

An der Vertikalstrebe 30 ist ein Aufnahmeblock 40 vertikal verschieblich gelagert. Der Aufnahmeblock 40 kann mittels eines Aktuators 41 in vertikaler Richtung entlang der Vertikalstrebe verschoben werden.

Durch die kombinierte translatorische Verschiebbarkeit der Vertikalstrebe 30 entlang der Längsstreben 21, 22 und des Aufnahmeblocks 40 entlang der Vertikalstrebe 30 kann der Aufnahmeblock in zwei Achsen in jeweils einem bestimmten, durch die Abmessungen der Längsstreben 21, 22 und der Vertikalstrebe 30 definierten Bereich verschoben und positioniert werden.

An dem Aufnahmeblock 40 ist ein Bügel 50 befestigt, der sich in horizontaler Richtung und senkrecht zu den Längsstreben durch den Aufnahmeblock 40 erstreckt. Der Bügel 50 ist außerhalb des Aufnahmeblocks beidseits nach unten gekrümmt und verläuft auf diese Weise seitlich außen von den Längsstreben 21, 22, zwei Abschnitte 51, 52 des Bügels erstrecken sich seitlich der Längsstreben 21, 22 nach senkrecht unten. Am Ende der sich senkrecht nach unten erstreckenden Abschnitte 51, 52 ist beidseits jeweils eine mehrgliedrige Gliederkette 61, 62 an den Bügelenden befestigt und erstreckt sich vertikal nach unten. Am Ende dieser jeweiligen Gliederkette 61, 62 ist eine Halterung 71, 72 angeordnet. Die Halterung 71, 72 ist dazu ausgebildet, um einen Ultraschallmesskopf 81, 82 aufzunehmen.

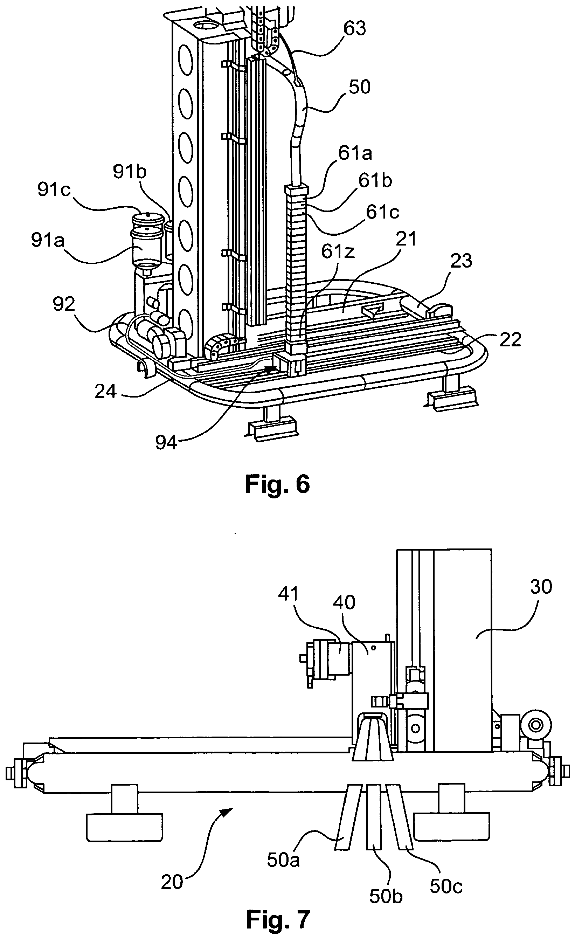

Fig. 6 zeigt die erfindungsgemäße Vorrichtung in einer Stellung, in welcher der Aufnahmeblock 40 an das obere Ende der Vertikalstrebe 30 verfahren ist. Wie ersichtlich, ist in dieser Position der Bügel 50, die Gliederketten 61, 62 und die Halterungen 71, 72 mit den daran angeordneten Ultraschallmessköpfen oberhalb des Rahmens 20 angeordnet und somit aus dem Aufenthaltsbereich 10 des Nutztiers herausgefahren. In dieser Position kann das Nutztier den Aufenthaltsbereich 10 betreten, ohne dass hierbei die Gefahr einer Beschädigung der Trächtigkeitsuntersuchungsvorrichtung besteht.

Durch Absenken des Aufnahmeblocks 40 entlang der Vertikalstrebe 30 und vorheriges, gleichzeitiges oder nachfolgendes Verfahren der Vertikalstrebe 30 entlang der Längsstreben 21 können die Halterungen 71, 72 mit den daran angeordneten Ultraschallmessköpfen 81, 82 an dem zu untersuchenden Nutztier im Bereich einer gewünschten Messposition positioniert werden. Zum Zwecke einer solchen Positionierung kann von dem Nutztier eine Umrissaufnahme von oben oder von seitlich aufgenommen werden, um hierdurch die gewünschte Messposition auf Grundlage einer Bildauswertung und vorgespeicherten Daten über entsprechende Messpositionen bestimmt werden. Zu diesem Zweck kann eine oder mehrere entsprechende Büdaufnahmevorrichtungen an der Halterung oder an anderer Position der erfindungsgemäßen Vorrichtung befestigt werden.

Fig. 7 zeigt den Aufnahmeblock 40 in einer abgesenkten Position. Wie ersichtlich, erstreckt sich in dieser abgesenkten Position der Bügel 50 nach unten in den Aufenthaltsbereich und die am Bügel 50 angeordneten Gliederketten 61, 62 verlaufen seitlich entlang des Körpers des Nutztieres.

Der Bügel 50 ist in dem Aufnahmeblock 40 schwenkbar gelagert und kann sich, wie in Fig. 7 durch die Positionen 50a,b,c gezeigt, durch Verschwenkung um eine horizontale Achse solcherart bewegen, dass die Halterungen 71, 72 am Ende der Gliederketten 61, 62 Vorwärts- oder Rückwärtsbewegungen des Nutztieres folgen können.

Wie insbesondere aus Fig. 6 zu erkennen, umfasst jede Gliederkette eine Mehrzahl von Kettengliedern 61a,b,c,... Jedes Kettenglied 61a,b,c,... ist mit dem ihm benachbarten Kettenglied schwenkbar verbunden. Diese schwenkbare Verbindung wird durch eine Achse bereitgestellt, die horizontal und parallel zu den Längsstreben 21, 22 verläuft. Die Schwenkachsen der Gliederketten 61, 62 liegen nahe der außen liegenden Seite der Kettenglieder im Inneren der Gliederkette.

Des Weiteren verläuft im Inneren der Gliederkette ein Seilzug 63, der sich von dem mit der Halterung verbundenen, unteren Kettenglied 61z ausgehend in Richtung des oberen Kettengliedes 61a erstreckt und von dort aus weiter durch den Innenraum des Haltebügels 50 verläuft. Im Bereich neben dem Aufnahmeblock 40 ist dieser Seilzug 63 aus dem Aufnahmebügel 50 herausgeführt und kann durch einen Aktuator 42 unter Zug gesetzt werden. Der Aktuator 42 setzt gleichzeitig die in der Gliederkette 61 und der Gliederkette 62 verlaufenden beiden Seilzüge unter Zug. Die Seilzüge verlaufen nahe der inneren Wand der Gliederketten 61, 62 und folglich beabstandet von den Schwenkachsen der Kettenglieder dieser Gliederketten. Hierdurch entsteht dann, wenn die Seilzüge unter Zug gesetzt werden, ein um diese Schwenkachsen wirkendes Moment, welches dazu führt, dass sich die Gliederketten solcherart verformen, dass sie eine nach außen konvexe und folglich nach innen konkave Form einnehmen und hierbei die Halterungen 71, 72 sich aufeinander zu bewegen.

Diese, durch Zug an den Seilzüge erzeugte Verformung der Gliederketten und Bewegung der Halterungen führt dazu, dass sich die Gliederketten an die Körperoberfläche des Nutztieres in einer nahezu druckfreien Weise anlegen und die Halterungen an die gewünschte Messposition solcherart heranführen, dass der in den Halterungen aufgenommene Ultraschallmesskopf 81, 82 sich auf die Hautoberfläche auflegt.

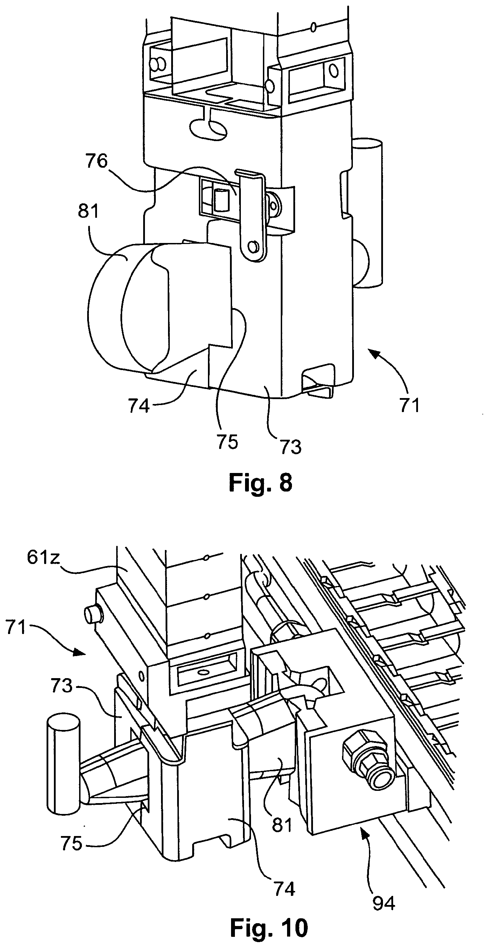

Fig. 8 zeigt die Halterung 71 mit darin eingesetztem Ultraschallmesskopf 81 in größerem Detail. Wie erkennbar ist, umfasst die Halterung 71 zwei aus Kunststoff gefertigte Blöcke 73, 74, die eine Ausnehmung 75 definieren. Die Blöcke 73, 74 sind mittels eines Kniehebelverschlusses 76 miteinander verbunden. Durch Öffnen des Kniehebelverschlusses 76 können die Blöcke 73, 74 voneinander getrennt werden, wodurch die Ausnehmung 75 geteilt wird und der Ultraschallmesskopf 81 in die Ausnehmung eingesetzt werden kann. Wird hierauf folgend der Kniehebelverschluss 76 wieder geschlossen, so ist der Ultraschallmesskopf 81 fest in der Halterung 71 verankert.

Fig. 9 zeigt ein System zur Benetzung der Ultraschallmessköpfe mit einem Kopplungsmedium. Dieses System ist Bestandteil der bevorzugten Ausführungsform der erfindungsgemäßen Trächtigkeitsuntersuchungsvorrichtung. Das System umfasst drei Behälter 91 a - c, welche einen Vorrat an Kopplungsmedium aufnehmen und aus denen dieses Kopplungsmedium über Schlauchleitungen 92, 93 zu Abgabevorrichtungen 94, 95 geleitet wird. Aus den Behältern 91a - c kann somit Kopplungsmedium in einen schlitzförmigen Zwischenraum der Abgabevorrichtungen 94, 95 gefördert werden. Um eine gleichmäßige und zugleich sparsame Benetzung der Ultraschallmessköpfe mit dem Kopplungsmedium zu erzielen, weisen die Abgabevorrichtungen 94, 95 jeweils einen beidseitig von dem Schlitz angeordneten Bürstenvorhang auf. Dieser Bürstenvorhang dient dazu, den jeweiligen Ultraschallmesskopf nach einer erfolgten Messung zu reinigen, bevor er mit Kopplungsmedium für eine neue Messung mit neuem Kopplungsmedium beschichtet wird. Das Kopplungsmedium wird zu diesem Zweck in den Zwischenraum eingefördert und so auf die Ultraschallmessköpfe aufgetragen.

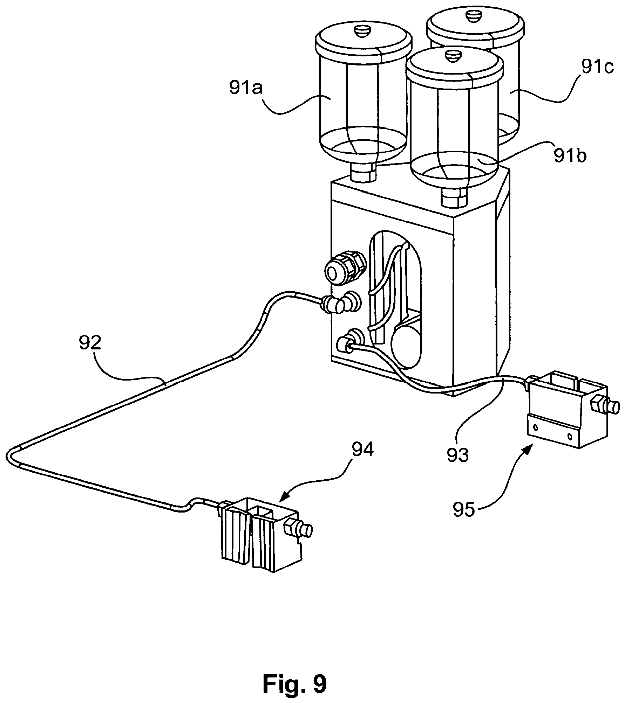

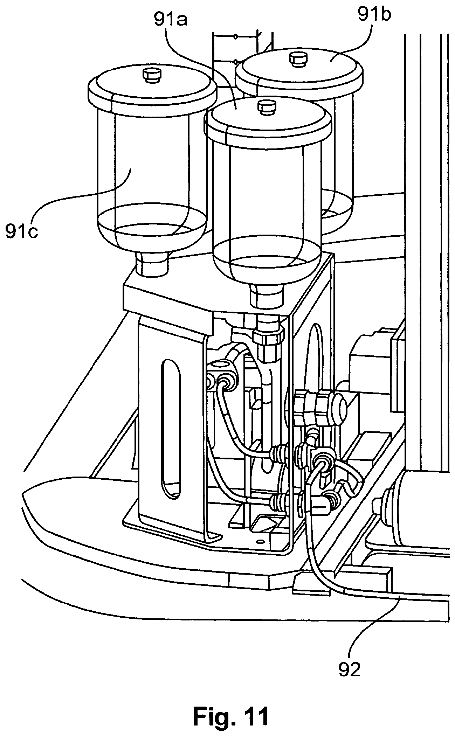

Fig. 10 zeigt den Ultraschallmesskopf 81 in einer in die Abgabevorrichtung 94 eingesetzten Position. Wie ersichtlich ist, berührt der Ultraschallmesskopf hierbei den Bürstenvorhang und wird solcherart gereinigt und danach mit dem Kopplungsmedium benetzt. Durch Eintreten des Ultraschallmesskopfes 81 in den Spalt in der Aufnahmevorrichtung 94 wird ein Sensor ausgelöst, der veranlasst, dass eine in Fig. 11 ersichtliche Schlauchpumpe für einen kurzen Zeitraum Kopplungsmedium durch die Schlauchleitungen 92, 93 fördert und hierdurch auf den Ultraschallmesskopf in die Bürstenvorhänge gelangt. Wird der Ultraschallmesskopf hiernach aus dem Aufnahmeschlitz wieder herausgezogen, so ist er für eine zuverlässige Messung ausreichend mit Kopplungsmedium benetzt.

Wie insbesondere aus Fig. 6 ersichtlich, sind die Abgabevorrichtungen 94, 95 im Bereich knapp oberhalb der Längsstreben 21, 22 montiert, so dass die Ultraschallmessköpfe 81, 82 in der angehobenen Position des Aufnahmeblocks 40 in die Abgabevorrichtungen 94, 95 eingeführt werden können.

## Patentansprüche

1. Vorrichtung zur Trächtigkeitsuntersuchung an einem Nutztier, umfassend:
- Eine Aufnahmevorrichtung, welche einen Aufenthaltsraum (10) für das zu untersuchende Nutztier definiert,
- Eine Halterung (71) mit einem darin aufgenommenen Ultraschallmesskopf (81), die mit einem relativ zu der Aufnahmevorrichtung beweglichen Gestell verbunden ist, und
- Eine Datenauswerteeinrichtung, die signaltechnisch mit dem Ultraschallmesskopf (81) verbunden oder verbindbar ist,
wobei die Datenauswerteeinrichtung ausgebildet ist, um mittels des Ultraschallkopfs (81) erfasste Messdaten durch Bilderkennung von Merkmalen zur Differenzierung einer Trächtigkeit des Nutztieres auszuwerten, **gekennzeichnet durch**
- eine zwei- oder dreidimensionale Bilderfassungseinrichtung zur Erfassung der Kontur oder eines Ausschnittes der Kontur des Nutztieres,
- eine mit der Bilderfassungseinrichtung signaltechnisch verbundene Bildauswertungseinheit, welche ausgebildet ist, um anhand eines Vergleichs der erfassten Kontur bzw. des erfassten Konturausschnitts mit gespeicherten anatomischen Daten einen Messpunkt oder Messbereich zu bestimmen, und
- eine mit der Bildauswertungseinheit signaltechnisch verbundene Messkopfhalterungssteuereinheit, welche mit einem oder mehreren Aktuatoren zur Bewegung der Halterung (71) signaltechnisch verbunden ist und ausgebildet ist, um die Halterung (71) so zu bewegen, dass der darin aufgenommene Ultraschallmesskopf (81) an den Messpunkt bzw. an den Messbereich geführt wird.

2. Vorrichtung nach Anspruch 1,
**dadurch gekennzeichnet, dass** die Aufnahmevorrichtung zwei den Aufenthaltsraum (10) des Nutztieres während der Untersuchung begrenzende Seitenwände (11, 12) aufweist und das Gestell eine Brücke aufweist, die ausgebildet ist, um entlang dieser Seitenwände (11, 12) horizontal bewegt zu werden.

3. Vorrichtung nach Anspruch 1 oder 2,
**dadurch gekennzeichnet, dass** die Aufnahmevorrichtung zwei den Aufenthaltsraum (10) des Nutztieres während der Untersuchung begrenzende Seitenwände (11, 12) aufweist und das Gestell eine Vertikalstrebe aufweist, mit der die Halterung (71) für eine vertikale Beweglichkeit des Ultraschallkopfs (81) verbunden ist, insbesondere solcherart, dass die Vertikalstrebe mit einer horizontal entlang der Seitenwände (11, 12) beweglichen Brücke verbunden ist und die Vertikalstrebe gemeinsam mit dem Ultraschallkopf (81) vertikal zu dieser Brücke beweglich ist oder der Ultraschallkopf (81) entlang der Vertikalstrebe vertikal beweglich ist.

4. Vorrichtung nach einem der vorhergehenden Ansprüche,
**dadurch gekennzeichnet, dass** die Halterung (71) am Ende einer mehrgliedrigen Gliederkette (61) befestigt ist, in der jeweils zwei benachbarte Kettenglieder (61a, 61b) um jeweils eine Kettengliedachse relativ zueinander verschwenkbar sind, die parallel zu der durch den Aufenthaltsraum (10) definierten Längsachse (100) des Nutztieres, insbesondere horizontal und parallel zu den den Aufenthaltsraum (10) begrenzenden Seitenwänden (11, 12) liegt.

5. Vorrichtung nach einem Anspruch 4,
**dadurch gekennzeichnet, dass** die Gliederkette (61) um zumindest eine Mehrzahl der Kettengliedachsen mittels eines Aktuators (42) verschwenkbar ist, vorzugsweise mittels eines innerhalb der Gliederkette (61) beabstandet von den Kettengliedachsen geführtem Zugseil, das mit einer aktuatorbetätigten Zugvorrichtung verbunden ist.

6. Vorrichtung nach einem der vorhergehenden Ansprüche,
**dadurch gekennzeichnet, dass**
- die Halterung (71) des Ultraschallmesskopfs (81) mit einem Aktuator (42) gekoppelt ist zur Positionierung des Ultraschallmesskopfs (81) an einer vorbestimmten Körperstelle des Nutztieres,
- das Gestell eine relative Beweglichkeit der Halterung (71) des Ultraschallmesskopfs (81) in zumindest einer Achse bereitstellt und
- eine Steuerung zur aktiven Positionierung und passiven Nachführung des in der Halterung (71) aufgenommenen Ultraschallmesskopfes (81) bereitgestellt ist, welche ausgebildet ist, um
∘ die Halterung (71) in einem Positionierungsmodus mittels eines oder mehreren Aktuatoren des Gestells so in Bezug auf die Aufnahmevorrichtung aktiv örtlich zu positionieren, wobei das Gestell ausschließlich aktuatorbetätigte Bewegungen der Halterung (71) ausführt, und
∘ die Halterung (71) in einem Messmodus in zumindest einer Achse so frei beweglich durch das Gestell so zu führen, dass die Halterung (71) Bewegungen des Nutztieres passiv folgen kann.

7. Vorrichtung nach einem der vorhergehenden Ansprüche, **gekennzeichnet durch** eine Benetzungsvorrichtung zur Zufuhr eines pastösen oder flüssigen Schallkopplungsmediums auf die Sende- und Empfangsfläche des Ultraschallkopfs (81), die Benetzungsvorrichtung umfassend:
- Einen Vorratsbehälter (91a) für das Schallkopplungsmedium,
- Eine Verbindungsleitung (92, 93) zur Leitung des Schallkopplungsmediums von dem Vorratsbehälter (91a) zu einer Austrittsöffnung, welche so angeordnet ist, dass aus der Austrittsöffnung austretendes Schallkopplungsmedium auf die Sende- und Empfangsfläche des Ultraschallkopfs (81) aufgetragen wird, und eine Fördervorrichtung zum Fördern des Schallkopplungsmediums von dem Vorratsbehälter (91a) zu der Austrittsöffnung.

8. Vorrichtung nach einem der vorhergehenden Ansprüche,
**dadurch gekennzeichnet, dass**
- die Aufnahmevorrichtung zwei den Aufenthaltsraum des Nutztieres während der Untersuchung begrenzende Seitenwände (11, 12) aufweist und das Gestell eine Brücke aufweist, die ausgebildet ist, um entlang dieser Seitenwände (11, 12) horizontal bewegt zu werden,
- eine Vertikalstrebe mit der Brücke verbunden ist,
- die Halterung (71) mit dem Ultraschallmesskopf (81) am Ende einer mehrgliedrigen Gliederkette (61) befestigt ist, in der jeweils zwei benachbarte Kettenglieder (61a, 61b) um jeweils eine Kettengliedachse relativ zueinander verschwenkbar sind, die parallel zu der durch den Aufenthaltsraum definierten Längsachse (100) des Nutztieres, insbesondere horizontal und parallel zu den den Aufenthaltsraum (10) begrenzenden Seitenwänden (11, 12) verläuft,
- die mehrgliedrige Kette (61) an der Vertikalstrebe in der Höhe verschieblich und um eine horizontale, quer zur Längsachse (100) des Nutztieres verlaufende Nachführachse verschwenkbar gelagert ist, und
- die Nachführachse vorzugsweise mittels eines Positionier- und Nachführmechanismus in einem Positionierungsmodus arretierbar ist und in einem Messmodus frei bewegbar ist.

9. Vorrichtung nach einem der vorhergehenden Ansprüche,
**gekennzeichnet durch** eine zweite Halterung (72) mit einem darin aufgenommenen zweiten Ultraschallmesskopf (82), wobei die zweite Haiterung (72) solcherart in Bezug auf die Halterung (71) mit dem Ultraschallmesskopf (81) angeordnet ist, dass der in der Halterung (71) und der zweiten Halterung (72) jeweils aufgenommene Ultraschallmesskopf (81) bzw. zweite Ultraschallmesskopf (82) einander zuweisen und solcherart beabstandet sind, dass sie gleichzeitig zwei gegenüberliegende Messpunkte oder Messbereiche am Nutztier erfassen können.

10. Vorrichtung nach einem der vorhergehenden Ansprüche,
**gekennzeichnet durch** eine an der Halterung (71) angeordnete horizontale Schwenkachse, mittels derer die Halterung (71) um eine horizontale und parallel zur Längsachse (100) des Nutztieres liegende Achse verschwenkt werden kann und weiter vorzugsweise **gekennzeichnet durch** einen zwischen Gestell und Halterung (71) mechanisch gekoppelten Schwenkaktuator, mittels dem die Halterung (71) um diese horizontale Schwenkachse verschwenkbar ist.

11. Verfahren zur Trächtigkeitsuntersuchung an einem Nutztier, umfassend:
- Aufnehmen des Nutztieres in einem umgrenzten Aufenthaltsraum (10),
- Vermessen der Tierkontur mittels einer zwei- oder dreidimensionalen Bilderfassu ngseinrichtu ng
- Auswerten der Tierkonturdaten und Bestimmen eines Messpunktes für eine Ultraschalluntersuchung anhand eines Vergleichs der Tierkonturdaten mit gespeicherten anatomischen Daten,
- Heranführen eines in einer Halterung (71) gehaltenen Ultraschallmesskopfs (81) an den Messpunkt durch Bewegen der Halterung (71) mittels eines Gestells relativ zu dem Aufenthaltsraum (10), Erfassen von Messdaten mittels des Ultraschallmesskopfs (81), und
- Auswerten der Messdaten durch Bilderkennung von Merkmalen die zur Differenzierung einer Trächtigkeit des Nutztieres herangezogen werden.

12. Verfahren nach Anspruch 11,
**dadurch gekennzeichnet, dass** das Vermessen der Tierkontur durch Abtasten der Kontur mittels eines Lasers erfolgt.

13. Verfahren nach Anspruch 11 oder 12,
**dadurch gekennzeichnet, dass** das Heranführen des Ultraschallmesskopfes (81) durch horizontales Verfahren einer Brücke, vertikales Verfahren einer an der Brücke gelagerten Ultraschallmesskopfhaltestrebe und/oder Anlegen einer mehrgliedrigen, den Ultraschallmesskopf (81) an einem Ende haltenden Gliederkette (61) an den Körper des Nutztieres erfolgt.

14. Verfahren nach einem Anspruch 13,
**dadurch gekennzeichnet, dass** die Gliederkette (61) an den Körper des Nutztieres angelegt wird, indem Kettengliedachsen, welche benachbarte Kettenglieder (61a, 61b) miteinander schnwenkbar koppeln, mittels eines Aktuators (42) verschwenkt werden, vorzugsweise indem ein innerhalb der Gliederkette (61) beabstandet von den Kettengliedachsen geführtes Zugseil mit dem Aktuator (42) unter Zug gesetzt wird.

15. Verfahren nach einem der vorhergehenden Ansprüche 11 bis 14,
**dadurch gekennzeichnet, dass**
- die Halterung (71) des Ultraschallmesskopfs (81) eine relative Beweglichkeit der Halterung (71) des Ultraschallmesskopfs (81) in zumindest einer Achse bereitstellt, und
- das Heranführen des Ultraschallmesskopfes (81) und Erfassen der Messdaten durch
∘ aktives Positionieren des Ultraschallmesskopfes (81) in einem Positionierungsmodus mittels eines oder mehreren Aktuatoren erfolgt, wobei das Gestell ausschließlich aktuatorbetätigte Bewegungen der Halterung (71) ausführt
∘ passives Nachführen des Ultraschallmesskopfes (81) in einem Messmodus um die zumindest eine Achse so dass der des Ultraschallmesskopf (81) den Bewegungen des Nutztieres passiv folgt.

## Claims

1. Device for examining a farm animal for gestation, comprising:
- a containment device defining a holding area (10) for the farm animal to be examined,
- a holder (71) having an ultrasonic measuring head (81) fitted in it which is connected to a frame that can be moved relative to the containment device,
and
- a data evaluation unit which is connected or can be connected to the ultrasonic measuring head (81) in a signal transmitting arrangement,
the data evaluation unit being configured to evaluate measurement data detected by means of the ultrasonic measuring head (81) by detecting features in images as a means of discerning a gestation of the farm animal,
**characterised by**
- a two- or three-dimensional image detection device for detecting a contour or a portion of the contour of the farm animal,
- an image evaluation unit connected to the image detection device in a signal transmitting arrangement, which is configured to determine a measuring point or measuring area on the basis of a comparison of the detected contour or detected contour portion with stored anatomical data,
and
- a measuring head holder control unit connected to the image evaluation unit in a signal transmitting arrangement which is connected in a signal transmitting arrangement to one or more actuators for moving the holder (71) and configured to move the holder (71) so that the ultrasonic measuring head (81) fitted in it is guided to the measuring point or into the measuring area.

2. Device as claimed in claim 1,
**characterised in that** the containment device has two side walls (11, 12) bounding the holding area (10) for the farm animal during the examination and the frame has a bridge which is designed to be moved horizontally along these side walls (11, 12).

3. Device as claimed in claim 1 or 2,
**characterised in that** the containment device has two side walls (11, 12) bounding the holding area (10) for the farm animal during the examination and the frame has a vertical strut to which the holder (71) is connected to enable a vertical movement of the ultrasonic measuring head (81), in particular such that the vertical strut is connected to a bridge that can be moved horizontally along the side walls (11, 12) and the vertical strut together with ultrasonic measuring head (81) is able to move vertically with respect to this bridge or the ultrasonic measuring head (81) is able to move vertically along the vertical strut.

4. Device as claimed in one of the preceding claims, **characterised in that** the holder (71) is attached to the end of a multilink link chain (61) in which two adjacent chain links (61a, 61b) can be respectively pivoted relative to one another about a chain link pin in each case lying parallel with the longitudinal axis (100) of the farm animal defined by the holding area (10), in particular horizontally to and parallel with the side walls (11, 12) bounding the holding area (10).

5. Device as claimed in claim 4,
**characterised in that** the link chain (61) can be pivoted about at least a plurality of the chain link pins by means of an actuator (42), preferably by means of a traction cable run inside the link chain (61) spaced at a distance apart from the chain link pins which is connected to an actuator-operated tensioning device.

6. Device as claimed in one of the preceding claims, **characterised in that**
- the holder (71) of the ultrasonic measuring head (81) is coupled with an actuator (42) for positioning the ultrasonic measuring head (81) on a predefined body point of the farm animal,
- the frame enables a relative movement of the holder (71) of the ultrasonic measuring head (81) in at least one axis and
- a controller is provided for actively positioning and passively tracking an ultrasonic measuring head (81) fitted in the holder (71)
which is configured
∘ in a positioning mode, to actively locally position the holder (71) relative to the containment device by means of one or more actuators of the frame, the frame effecting exclusively actuator-operated movements of the holder (71), and
∘ in a measuring mode, to guide the holder (71) to enable it to move freely in at least one axis by means of the frame such that the holder (71) is able to follow movements of the farm animal passively.

7. Device as claimed in one of the preceding claims, **characterised by** a wetting device for feeding a pasty or liquid acoustic coupling medium to the transmitter and receiver surface of the ultrasonic head (81),
the wetting device comprising:
- a supply container (91a) for the acoustic coupling medium,
- a connecting pipe (92, 93) for directing the acoustic coupling medium from the supply container (91a) to an outlet orifice which is disposed so that acoustic coupling medium leaving the outlet orifice is applied to the transmitter and receiver surface of the ultrasonic head (81), and a conveying device for conveying the acoustic coupling medium from the supply container (91a) to the outlet orifice.

8. Device as claimed in one of the preceding claims, **characterised in that**
- the containment device has two side walls (11, 12) bounding the holding area for the farm animal during the examination and the frame comprises a bridge configured to be moved horizontally along these side walls (11, 12),
- a vertical strut is connected to the bridge,
- the holder (71) with the ultrasonic measuring head (81) is attached to the end of a multilink link chain (61) in which two adjacent chain links (61a, 61b) can be pivoted respectively relative to one another about a chain link pin in each case and which extends parallel with the longitudinal axis (100) of the farm animal defined by the holding area, in particular horizontally to and parallel with the side walls (11, 12) bounding the holding area (10),
- the multilink chain (61) is mounted so that it can be moved across the height of the vertical strut and
- pivoted about a horizontal tracking shaft extending transversely to the longitudinal axis (100) of the farm animal, and
- the tracking shaft can preferably be locked by means of a positioning and tracking mechanism in a positioning mode and is able to move freely in a measuring mode.

9. Device as claimed in one of the preceding claims, **characterised by** a second holder (72) having a second ultrasonic measuring head (82) fitted in it, the second holder (72) being disposed such relative to the holder (71) with the ultrasonic measuring head (81) that the ultrasonic measuring head (81) and/or second ultrasonic measuring head (82) fitted in the holder (71) and the second holder (72) respectively face one another and are spaced apart such that they are able to detect two oppositely lying measuring points or measuring areas on the farm animal simultaneously.

10. Device as claimed in one of the preceding claims, **characterised by** a horizontal pivot pin disposed on the holder (71) by means of which the holder (71) can be pivoted about a horizontal axis lying parallel with the longitudinal axis (100) of the farm animal and preferably further **characterised by** a pivot actuator mechanically coupled between the frame and holder (71) by means of which the holder (71) can be pivoted about this horizontal pivot pin.

11. Method for examining a farm animal for gestation, comprising:
- holding the farm animal in an enclosed holding area (10),
- measuring the animal contour by means of a two-or three-dimensional image detection device,
- evaluating the animal contour data and determining a measuring point for an ultrasound examination based on a comparison of the animal contour data with stored anatomical data,
- moving an ultrasonic measuring head (81) fitted in a holder (71) to the measuring point by moving the holder (71) relative to the holding area (10) by means of a frame,
- detecting measurement data by means of the ultrasonic measuring head (81), and
- evaluating the measurement data by detecting features in images which are applied as a means of discerning a gestation of the farm animal.

12. Method as claimed in claim 11,
**characterised in that** the animal contour is measured by scanning the contour by means of a laser.

13. Method as claimed in claim 11 or 12,
**characterised in that** the ultrasonic measuring head (81) is moved by moving a bridge horizontally, moving an ultrasonic measuring head retaining strut mounted on the bridge vertically and/or placing a multilink link chain (61) with the ultrasonic measuring head (81) attached to one end in contact with the body of the farm animal.

14. Method as claimed in claim 13,
**characterised in that** the link chain (61) is placed in contact with the body of the animal by pivoting chain link pins pivotably coupling adjacent chain links (61a, 61b) with one another by means of an actuator (42), preferably by placing a traction cable guided inside the link chain (61) at a distance apart from the chain link pins under tension by means of an actuator (42).

15. Method as claimed in one of preceding claims 11 to 14, **characterised in that**
- the holder (71) of the ultrasonic measuring head (81) enables a relative movement of the holder (71) of the ultrasonic measuring head (81) in at least one axis, and
- the ultrasonic measuring head (81) is moved and the measurement data detected by
∘ in a positioning mode, actively positioning the ultrasonic measuring head (81) by means of one or more actuators, the frame effecting exclusively actuator-operated movements of the holder (71),
∘ in a measuring mode, passively tracking the ultrasonic measuring head (81) about the at least one axis such that the ultrasonic measuring head (81) follows the movements of the farm animal passively.

## Revendications

1. Dispositif de détection de gravidité sur un animal d'élevage, comprenant :
- un dispositif de réception, lequel définit un espace de séjour (10) pour l'animal d'élevage à examiner,
- un support (71) avec une tête de mesure à ultrasons (81) reçue dans celui-ci, qui est relié à un bâti mobile par rapport au dispositif de réception, et
- un appareil d'évaluation de données qui est relié ou peut être relié par la technique de signalisation à la tête de mesure à ultrasons (81),
dans lequel l'appareil d'évaluation de données est réalisé pour évaluer des données de mesure détectées au moyen de la tête à ultrasons (81) par détection d'image de caractéristiques de différenciation d'une gravidité de l'animal d'élevage,
**caractérisé par**
- un appareil de détection d'image à deux ou trois dimensions pour la détection du contour ou d'une section du contour de l'animal d'élevage,
- une unité d'évaluation d'image reliée par la technique de signalisation à l'appareil de détection d'image, laquelle est réalisée pour déterminer un point de mesure ou une zone de mesure à l'aide d'une comparaison du contour détecté ou de la section de contour détectée avec des données anatomiques enregistrées, et
- une unité de commande de support de tête de mesure reliée par la technique de signalisation à l'unité d'évaluation d'image, laquelle est reliée par la technique de signalisation à un ou plusieurs actionneurs pour le déplacement du support (71), et est réalisée pour déplacer le support (71) de telle sorte que la tête de mesure à ultrasons (81) reçue dans celui-ci est guidée au point de mesure ou à la zone de mesure.

2. Dispositif selon la revendication 1,
**caractérisé en ce que** le dispositif de réception présente deux parois latérales (11, 12) délimitant l'espace de séjour (10) de l'animal d'élevage pendant l'examen et le bâti présente un pont, qui est réalisé pour être déplacé horizontalement le long de ces parois latérales (11, 12).

3. Dispositif selon la revendication 1 ou 2,
**caractérisé en ce que** le dispositif de réception présente deux parois latérales (11, 12) délimitant l'espace de séjour (10) de l'animal d'élevage pendant l'examen et le bâti présente un montant vertical, auquel le support (71) est relié pour une mobilité verticale de la tête à ultrasons (81), en particulier de telle manière que le montant vertical est relié à un pont horizontalement mobile le long des parois latérales (11, 12) et le montant vertical est mobile conjointement avec la tête à ultrasons (81) verticalement par rapport à ce pont ou la tête à ultrasons (81) est verticalement mobile le long du montant vertical.

4. Dispositif selon l'une quelconque des revendications précédentes,
**caractérisé en ce que** le support (71) est fixé à l'extrémité d'une chaîne de maillons à plusieurs maillons (61), dans laquelle respectivement deux maillons de chaîne (61a, 61b) adjacents sont aptes à pivoter l'un par rapport à l'autre autour de respectivement un axe de maillon de chaîne, qui se trouve parallèlement à l'axe longitudinal (100) de l'animal d'élevage défini par l'espace de séjour (10), en particulier horizontalement et parallèlement aux parois latérales (11, 12) délimitant l'espace de séjour (10).

5. Dispositif selon une revendication 4,
**caractérisé en ce que** la chaîne de maillons (61) est apte à pivoter autour d'au moins une pluralité des axes de maillon de chaîne au moyen d'un actionneur (42), de préférence au moyen d'un câble de traction guidé à l'intérieur de la chaîne de maillons (61) à distance des axes de maillon de chaîne, qui est relié à un dispositif de traction actionné par actionneur.

6. Dispositif selon l'une quelconque des revendications précédentes,
**caractérisé en ce que**
- le support (71) de la tête de mesure à ultrasons (81) est couplé avec un actionneur (42) pour le positionnement de la tête de mesure à ultrasons (81) à un endroit prédéterminé du corps de l'animal d'élevage,
- le bâti met à disposition une mobilité relative du support (71) de la tête de mesure à ultrasons (81) dans au moins un axe et
- une commande est mise à disposition pour le positionnement actif et la poursuite passive de la tête de mesure à ultrasons (81) reçue dans le support (71), laquelle est réalisée pour
- positionner ainsi le support (71) dans un mode de positionnement au moyen d'un ou plusieurs actionneurs du bâti de manière localement active par rapport au dispositif de réception, dans lequel le bâti réalise exclusivement des mouvements actionnés par actionneur du support (71), et
- guider ainsi le support (71) dans un mode de mesure dans au moins un axe de manière librement mobile par le bâti de sorte que le support (71) puisse suivre passivement des mouvements de l'animal d'élevage.

7. Dispositif selon l'une quelconque des revendications précédentes,
**caractérisé par** un dispositif de mouillage pour l'amenée d'un milieu de couplage sonore pâteux ou liquide sur la surface d'émission et de réception de la tête à ultrasons (81), le dispositif de mouillage comprenant :
- un réservoir (91a) pour le milieu de couplage sonore,
- une conduite de liaison (92, 93) pour la conduite du milieu de couplage sonore du réservoir (91a) à une ouverture de sortie, laquelle est agencée de telle sorte que du milieu de couplage sonore sortant de l'ouverture de sortie est appliqué sur la surface d'émission et de réception de la tête à ultrasons (81), et un dispositif de transport pour le transport du milieu de couplage sonore du réservoir (91a) à l'ouverture de sortie.

8. Dispositif selon l'une quelconque des revendications précédentes,
**caractérisé en ce que**
- le dispositif de réception présente deux parois latérales (11, 12) délimitant l'espace de séjour de l'animal d'élevage pendant l'examen et le bâti présente un pont, qui est réalisé pour être déplacé horizontalement le long de ces parois latérales (11, 12),
- un montant vertical est relié au pont,
- le support (71) est fixé à la tête de mesure à ultrasons (81) à l'extrémité d'une chaîne de maillons à plusieurs maillons (61), dans laquelle respectivement deux maillons de chaîne (61a, 61b) adjacents sont aptes à pivoter l'un par rapport à l'autre autour de respectivement un axe de maillon de chaîne, qui s'étend parallèlement à l'axe longitudinal (100) de l'animal d'élevage défini par l'espace de séjour, en particulier horizontalement et parallèlement aux parois latérales (11, 12) délimitant l'espace de séjour (10).
- la chaîne à plusieurs maillons (61) est logée de manière mobile en hauteur au niveau du montant vertical et de manière pivotante autour d'un axe de poursuite s'étendant transversalement à l'axe longitudinal (100) de l'animal d'élevage, et
- l'axe de poursuite peut être arrêté de préférence au moyen d'un mécanisme de positionnement et de poursuite dans un mode de positionnement et est librement mobile dans un mode de mesure.

9. Dispositif selon l'une quelconque des revendications précédentes,
**caractérisé par** un deuxième support (72) avec une deuxième tête de mesure à ultrasons (82) reçue dans celui-ci, dans lequel le deuxième support (72) est disposé par rapport au support (71) avec la tête de mesure à ultrasons (81) de telle manière que la tête de mesure à ultrasons (81) ou la deuxième tête de mesure à ultrasons (82) reçue respectivement dans le support (71) et le deuxième support (72) sont affectées l'une à l'autre et espacées de telle manière qu'elles peuvent détecter en même temps deux points de mesure ou zones de mesure opposées sur l'animal d'élevage.

10. Dispositif selon l'une quelconque des revendications précédentes,
**caractérisé par** un axe de pivotement horizontal disposé au niveau du support (71), au moyen duquel le support (71) peut pivoter autour d'un axe horizontal et situé parallèlement à l'axe longitudinal (100) de l'animal d'élevage et plus préférablement **caractérisé par** un actionneur de pivotement couplé mécaniquement entre le bâti et le support (71), au moyen duquel le support (71) peut pivoter autour de cet axe de pivotement horizontal.

11. Procédé de détection de gravidité sur un animal d'élevage, comprenant :
- la réception de l'animal d'élevage dans un espace de séjour (10) délimité,
- la mesure du contour de l'animal au moyen d'un appareil de détection d'image à deux ou trois dimensions,
- l'évaluation des données de contour d'animal et la détermination d'un point de mesure pour un examen par ultrasons à l'aide d'une comparaison des données de contour d'animal avec des données anatomiques enregistrées,
- l'approche d'une tête de mesure à ultrasons (81) maintenue dans un support (71) du point de mesure par déplacement du support (71) par rapport à l'espace de séjour (10) au moyen d'un bâti,
- la détection de données de mesure au moyen de la tête de mesure à ultrasons (81), et
- l'évaluation des données de mesure par détection d'image de caractéristiques qui sont prises en compte pour la différenciation d'une gravidité d'un animal d'élevage.

12. Procédé selon la revendication 11,
**caractérisé en ce que** la mesure du contour d'animal se fait par balayage du contour au moyen d'un laser.

13. Procédé selon la revendication 11 ou 12,
**caractérisé en ce que** l'approche de la tête de mesure à ultrasons (81) se fait par déplacement horizontal d'un pont, déplacement vertical d'un montant de support de tête de mesure à ultrasons logé au niveau du pont et/ou application d'une chaîne de maillons (61) à plusieurs maillons, maintenant la tête de mesure à ultrasons (81) à une extrémité, sur le corps de l'animal d'élevage.

14. Procédé selon une revendication 13,
**caractérisé en ce que** la chaîne de maillons (61) est appliquée sur le corps de l'animal d'élevage, par le fait que les axes de maillon de chaîne, lesquels couplent de manière pivotante l'un à l'autre des maillons de chaîne (61a, 61b) adjacents, sont mis en pivotement au moyen d'un actionneur (42), de préférence par le fait qu'un câble de traction guidé à l'intérieur de la chaîne de maillons (61) à distance des axes de maillon de chaîne est mis sous tension avec l'actionneur (42).

15. Procédé selon l'une quelconque des revendications précédentes 11 à 14,
**caractérisé en ce que**
- le support (71) de la tête de mesure à ultrasons (81) met à disposition une mobilité relative du support (71) de la tête de mesure à ultrasons (81) dans au moins un axe, et
- l'approche de la tête de mesure à ultrasons (81) et la détection des données de mesure se fait par
- positionnement actif de la tête de mesure à ultrasons (81) dans un mode de positionnement au moyen d'un ou plusieurs actionneurs, dans lequel le bâti réalise exclusivement des mouvements actionnés par actionneur du support (71)
- poursuite passive de la tête de mesure à ultrasons (81) dans un mode de mesure autour de l'au moins un axe de sorte que la tête de mesure à ultrasons (81) suit passivement les mouvements de l'animal d'élevage.
